# EUROPEAN PATENT APPLICATION

(11) **EP 0 781 853 A2**
(43) Date of publication of application: **02.07.1997**
(21) Application number: 96308898.4
(22) Date of filing: 09.12.1996
(51) Int. Cl.: C12Q 1/68

(54) **Method for measuring nucleic acids**

(30) Priority: 12.12.1995 US 570998
(71) Applicant: ELI LILLY AND COMPANY, Indianapolis, Indiana 46285 (US)
(72) Inventor: Basinski, Margret Barbara, Indinapolis, Indinapolis, Indiana 46219 (US); Kahl, Steven Dean, Fishers, Indiana 46038 (US); Reifel-Miller, Anne Elizabeth, Indianapolis, Indiana 46208 (US); Schoner, Brigitte Elisabeth, Monrovia, Indiana 46157 (US); Sittampalam, Sitta Gurusingham, Indianapolis, Indiana 46256 (US)
(74) Representative: Hudson, Christopher Mark

(57) **Abstract**

This invention describes methods for using peptide nucleic acid (PNA) and/or deoxy nucleotide guanidine (DNG) derivatives as probes to quantify specific RNA or DNA sequences, especially those associated with gene function. The invention eliminates the need to remove unbound probes and offers advantages relating to specificity, sensitivity, and reproducibility. The claimed methods are particularly useful in high volume screens.

## Description

The present invention belongs to the field of molecular biology as it applies to methods of assaying specific RNA or DNA sequences associated with gene function. The methods employ peptide nucleic acid (PNA) oligomers or deoxy nucleotide guanidine (DNG) derivatives in conjunction with scintillation or fluorescence reporting technology.

PNA oligomers and DNG derivatives are synthetic DNA analogs that have been shown to tightly bind DNA and RNA in a sequence specific manner (Nielson, P.E. *et al.* (1991) *Science,* 254, 1497-1500). Substituting the ribose phosphate backbone of natural DNA with a different backbone resembling that found in proteins has furnished molecular biologists with a new synthetic form of DNA. These new oligomers represent versatile mimetics for oligonucleotides as they are largely resistant to nucleases and proteases and can be readily labeled with fluorescent tags, radioisotopes, biotin, phosphatases, and other reporter groups.

PNAs and DNGs have been used as substitutes for oligonucleotides in molecular biology procedures and have been shown to bind DNA and RNA tighter and are less sensitive to salt concentration effects than DNA (Egholm, M. *et al.* (1993) *Nature* 365, 566-568). Therefore, short radiolabeled PNAs or DNGs can be used to probe and quantitate genetic messages in otherwise conventional DNA methodologies.

To date, PNAs and DNGs have not been used as probes in scintillation proximity assays (SPA). Nor have they been used in conjunction with fluorescence polarization assays where bound probes emit a signal which can be distinguished from that emitted from unbound probes .

SPA technology is well known in the art as evidenced by U.S. Patent Number 4,568,649, herein incorporated by reference. Briefly, this technology allows one to detect minuscule amounts of a reactant in a test sample using beads or support structures that have been impregnated with a scintillant and coated with a ligand that specifically binds to the substance of interest (reactant). The impregnated beads are then placed in a solution containing radio-labeled reactant, or a radio-labeled agent that specifically binds the reactant. The directly or indirectly labeled reactant which binds to the ligand on the surface of the SPA bead will be brought in close enough proximity to the beads to activate the scintillant to produce detectable energy. Directly or indirectly labeled reactant which fails to bind to the ligand on the surface of the SPA bead generally will be too far removed from the scintillant to excite any emitting energy. Thus, the amount of energy emitted is indicative of the amount of reactant present in the test sample. This technology has also been adapted for use in a competitive mode analogous to competitive radio-immuno-assays.

SPA technology has been adapted to quantitate genetic messages only following a polymerase chain reaction whereby radiolabeled nucleotides are incorporated into a growing chain complementary to a strand of target DNA using a capture probe (Schoenfeld, A. and Luqmani, Y.A. (1995) *Anal. Biochem.* 228, 164-167). Prior to this invention, SPA has not been adapted for use with capture and reporter probes in tandem.

In one embodiment, the present invention offers a convenient means for overcoming the problem of removing unbound probes prior to adding SPA beads. In all embodiments, the present invention provides other benefits relating to specificity, sensitivity, and reproducibility.

The present invention claims a method for quantitating genetic material which comprises placing in an aqueous suspension of genetic material, a PNA or DNG reporter probe tagged with a reporting group and a PNA or DNG capture probe tagged with a first member of a binding pair such that each probe is in molar excess of genetic material to be quantitated under conditions that permit hybridization. This step is followed by exposing the above suspension to a capture surface impregnated with a scintillant and tagged with a second member of the aforementioned binding pair under conditions that permit the binding pair members to bind to each other. Lastly the amount of reporter probe that is complexed with the capture probe, the capture surface, and the genetic material to be measured is quantitated using conventional scintillation proximity methods.

A second method for quantitating genetic material is claimed which comprises placing, in an aqueous suspension of genetic material, a reporter probe tagged with a fluorophore in molar excess of genetic material to be quantitated under conditions that permit hybridization. Quantitating the amount of reported probe that has hybridized with the genetic material to be measured is accomplished using fluorescence polarization methodology.

The present invention uses PNA and/or DNG derivatives as probes to quantify specific RNA or DNA sequences of any kind. These nucleic acid sequences include ssDNA, mRNA, tRNA, and rRNA. Methods for synthesizing PNA and DNG derivatives having a particular sequence of bases are well known in the art.

To practice the presently claimed invention, two probes are prepared which have a base sequence complementary to two distinct regions of the genetic material of interest. The first probe (reporter probe) is labeled with any one of the following reporter groups which represents merely an illustrative list of reporter groups that is consistent with the present invention: radioisotopes, fluorescent molecules, or enzymes. The second probe (capture probe) is labeled with a molecule or group that represents part of a binding pair. A binding pair is simply two molecules which are capable of selectively binding to each other. Illustrative examples of binding pairs include antigen:antibody, hapten:antibody, biotin:avidin, biotin:streptavidin, and biotin:neutravidin. Many other binding pairs, some of which may have indirect coupling schemes, are known in the art and are consistent with the invention. For purposes of this invention, biotin:streptavidin is the preferred binding pair and biotin is preferably bound to the capture probe.

In PNA probes, negatively charged phosphate groups are replaced by an uncharged 2-aminoethyl backbone which hybridizes to RNA and ssDNA molecules with higher affinity than corresponding DNA or RNA probes. The DNG probes contain positively charged guanidinium groups which enhance the base pairing via electrostatic interaction with the negatively charged phosphate groups in RNA or ssDNA. In both cases, the hybrids have much higher melting temperatures than their complexes with RNA or DNA probes. This higher affinity and the use of sensitive labels allow for the design of sensitive assays to measure gene products of interest.

In one embodiment, the reporter and capture probes are added simultaneously to a solution containing the genetic material. Following hybridization, complexes arise that contain the two probes hybridized to the DNA or RNA of interest. These tertiary complexes are then captured on beads or plates coated with capture molecules that bind to their respective binding partner.

For example, a PNA probe labeled with ¹²⁵I and designed to hybridize to one region of a particular mRNA is mixed with a biotin labeled PNA probe designed to hybridize to a different region of the same mRNA. This mixture is then added to a solution containing the mRNA and are allowed to hybridize. The solution is then exposed to a capture surface such as avidin coated microtiter wells and allowed to incubate. All of the molecules that have not been captured are then removed and the bound signal is quantified using standard radionuclide counting methods. Thus the only way that the ¹²⁵I label can be captured and counted is by hybridizing to the mRNA which also must be hybridized to the capture probe which in turn must bind to the capture surface.

In another embodiment, the invention can avoid the need to remove any unbound components when SPA methodologies are used. In this mode, the reporter probe and capture probes remain the same. The capture surface however is modified by incorporating a scintillant. Preferably the capture surface is a scintillant-impregnated bead. Therefore, the only way that the ¹²⁵I label on the reporter probe is brought in close enough proximity to excite the scintillant is by specific hybridization to the mRNA of interest, the reporter probe and the capture probe. Thus, the label can be directly quantified without the need to separate the complex from the reaction media because unbound reporter probes will not remain in close enough proximity to the scintillation-doped bead to excite it. The latter well-known reporting technology is collectively known as scintillation proximity assays. Scintillant doped beads and coated plates having immobilized capture molecules are commercially available.

Assay specificity is determined by the number and sequence of the nucleotide bases on the reporter and capture probes. It has been determined that the size of the probes may vary anywhere from a few base pairs to relatively large fragments; however, the shorter the probe, the less specificity is seen. As in analogous molecular biology techniques, optimal probe length ranges from 12-18 bases in length which corresponds to the inverse complementary sequence of the 5' region of the genetic material to be assayed.

In addition, both the reporter and capture probes contribute to the specificity of the assay by recognizing two different regions of the same genetic material. Research leading to the present invention showed that both probes hybridized to a target DNA in the presence of several fold excess of non-specific yeast RNA. An additional source of specificity comes from the fact that a mismatch in PNA-RNA duplexes is more destabilizing than in DNA-RNA duplexes. Hence, the PNA probes have provided benefits over conventional DNA or RNA probes.

PNA or DNG reporter probes are synthesized with a lysine or tyrosine residue at the 3' or 5' ends using well established, published procedures. The lysine and tyrosine residues are then labeled with either ¹²⁵I or ³H using standard radilolabeling techniques. ¹⁴C, ³⁵S, ³²P, or ³³P isotopes can also be used as labels depending on the detection technology to be employed. Multiple tyrosine and lysine residues can be used to increase specific activity of the labeled reporter probe. Procedures are also available to label the reporter probes with fluorescent and enzyme labels. These probes allow for non-isotopic detection techniques to be employed in the assay.

The capture probes are preferably tagged with an antigen, hapten or biotin and/or biotin derivatives using well established protocols. Biotin tagged probes are more preferred.

The use of PNA oligomers offers advantages in sensitivity, specificity and ease of testing large numbers of samples compared to more conventional methods employing DNA or RNA probes. Because the PNA backbone is neutral, there is no charge repulsion in the PNA-RNA or PNA-DNA duplexes. Therefore, the PNA duplexes are more stable than a DNA-RNA duplex (Egholm, M. *et al.* (1993) *Nature* 365, 566-568). In addition, the polyamide backbone of the PNA probes are not hydrolyzed by known nucleases or proteases resulting in excellent in-vivo and in-vitro stability. This resistance to enzymatic degradation and increased stability of the PNA-DNA and PNA-RNA duplexes has unique advantages over the use of DNA and RNA probes. PNA probes bind RNA and in very low salt concentrations and in the absence of Mg⁺⁺. Hence assay conditions can be selected so that PNA probes can be used to capture RNA selectively in the presence of DNA in the sample. DNG probes offer similar advantages since they are also resistant to enzymatic degradation.

In cell-based assays, the reporter and capture probes can be added into the lysis buffer. Components of lysis buffers such as glycerol, NP-40 and EDTA were found to have no effect on the hybridization of these probes to the target gene product. Capture of these complexes onto SPA beads or plates and other solid phase reagents is also not affected by the presence of buffer components. Therefore, these probes are quite effective in assays involving complex matrices.

When fluorescently labeled reporter probes are used, the hybridized complex can be captured on latex beads coated with the capture molecules. These latex beads can be washed and the fluorescence signal on the beads can then be quantified by measuring the solid-phase fluorescence in commercially available instruments such as the Screen Machine manufactured by IDEX Inc. In this instrument, surface concentrated fluorescence can be measured in specially designed 96-well plates that enable the filtration of excess reagents, wash steps, and measurement of fluorescent probe captured on the bead.

In yet another embodiment, fluorescence polarization can be used to measure the extent of binding without the need to separate the free probes from the bound probes. Fluorescence polarization measures changes in molecular volume of a labeled probe when it hybridizes to an RNA or a ssDNA target of higher molecular size. When a labeled probe with approximately 12-18 bases (∼3-5,000 daltons) binds to a target RNA or ssDNA such that the net change in molecular weight is greater than 25,000 daltons, significant increase in the polarization signal is observed. Since almost all gene products are several thousand kilobases in size, significant increase in polarization signal will be observed in all cases. An added advantage of using fluorescent polarization is that the measurement can be made in an homogeneous solution without the need for a capture probe. Hence only a single reporter probe is required to specifically quantify the genetic material.

The assay procedures described above can be easily configured in 96-well assay plate format for high throughput screens, and in secondary and tertiary assays. Therefore, chemical entities that alter the levels of genetic messages of pharmacological interest in specially designed *in vivo* or *in vitro* assay systems employing prokaryotic and/or eukaryotic cells can be screened readily in high volume. The unique stability and specificity of the modified nucleic acid probes, combined with sensitive detection systems using radioisotopes, fluorescent and enzyme labels offer a novel tool to investigate chemical compounds that may effect genetic alterations.

By way of illustration, the following examples are provided to help describe how to make and practice the various embodiments of the invention. These example are in no way meant to limit the scope of the invention.

### EXAMPLE 1

### Detection of Synthetic Oligonucleotide DNA using SPA

The present invention was used to detect synthetic oligonucleotide DNA molecules with a scintillation proximity bead-based assay. Specific reagents required for implementation included-coated SPA beads (obtained from Amersham International, Plc.), a PNA capture probe tagged with biotin at the 5' end, a PNA reporter probe having a ³H lysine attached to the 3' end, and a synthetic DNA oligonucleotide having complementary regions to both PNA probes. The PNA probes were custom ordered from Perseptive Biosystems and had base pair sequences that were complementary to the putative sequence for insulin mRNA. The synthetic oligonucleotide DNA was obtained commercially (Genosys) or prepared in-house. Sequences of these reagents are shown below followed by the procedure for labeling the PNA-Lys probe with ³H and the SPA measurement of oligonucleotide DNA.

| **Reagent** | **Sequence** |
|---|---|
| Biotin-PNA | 5'-Bio-OO-ACA-GGG-CCA-TGT-TGA-AAC-CONH2 |
| PNA-Lys | 5'-H-AAT-RAC-CTG-CTT-GCT-GAT-G-O-Lys |
| Oligonucleotide | 5'-CAAAAAAACCATCAGCAAGCAGGTYATTGTTTCAAC |
| DNA | ATGGCCCTGTGGATGCG **(SEQ ID NO:1)** |

### Preparation of PNA Reagents:

Lyophilized PNA-Lys (100 nmoles) was reconstituted in 0.2 ml 0.1% TFA (v/v) in DEPC-treated water (diethyl pryocarbonate treated to remove RNAses, DNAases and proteases). Concentration was checked by pipetting 1 µl into 999 µl of water and measuring at 260 nm in a spectrophotometer. According to the manufacturer's technical literature that was provided, 1 OD₂₆₀ nm is equal to approximately 26 µg of PNA. Similarly, the lyophilized Biotin-PNA (80 nmoles) was dissolved in 0.2 ml 0.1% TFA as described above. The oligonucleotide DNA (11.7 nmoles) was dissolved in 0.1 ml DEPC-treated water. All samples were aliquoted and stored frozen at -20°C.

### Preparation of ³H-labeled PNA:

The reaction to label the PNA-Lys molecule utilized a chemical reaction between a hydroxy succinimide moeity and the epsilon-amine of the attached lysine residue of the PNA. All steps were performed in polypropylene tubes. 118 µl of Succinimidyl Propionate, N-[Propionate-2,3-³H] (³H NSP, NEN/DuPont, 58.5 Ci/mmol) was evaporated completely under a stream of dry gaseous nitrogen. 2 µl (1 nmole) of dissolved PNA-Lys was added to 8 µl of 0.2 M Hepes, pH 7.5. This mixture was placed into the tube containing the dried ³H NSP. This resulted in a 1 to 2 molar ratio of PNA-Lys to ³H NSP. The sample was incubated on ice for 30 min followed by overnight incubation at 4°C. The reaction was quenched with 10 µl of 1M Tris®-HCl, pH 8.5 and incubated an additional 30 min at 4°C. BSA (20 µl of 1 mg/ml stock) was added to the sample as a carrier protein and the sample was desalted on a 1.5 ml Sephadex® G-15 column (Pharmacia) pre-equilibrated with 1 column volume of Dulbecco's phosphate buffered saline (D-PBS, without calcium or magnesium, Gibco/BRL) containing 1 mg/ml BSA followed by 5 column volumes of D-PBS. Following addition of the sample to the top of the column packing, the column was eluted with D-PBS. Two-drop fractions (-45 µl/drop) were collected. The fractionation range for Sephadex® G-15 allowed molecules larger than 1500 Da to pass through the column into the void volume. Molecules smaller than 1500 Da were retained by the column as they passed through the pores of the packing material. Therefore, the covalently ³H-labeled PNA (MW > 5000) was not retained by the column packing and appeared in the first peak. Unbound ³H-NSP appeared in the second peak of radioactivity. Radioactivity in the individual fractions was analyzed by counting 1 µl of each fraction in 5 ml of scintillation cocktail. Peak fractions were combined resulting in an approximate specific activity (assuming 100% recovery of the PNA) of 27 Ci/mmol. This material was aliquoted and stored frozen at -20°C.

### Scintillation Proximity Assay:

To demonstrate the principle of this invention, an SPA assay was performed with different conditions representing one or more of the assay components being deleted from the incubation mixture. In all conditions, streptavidin SPA beads were included as well as ³H-labeled PNA at three different levels.

Assays were performed in 96-well clear-bottom polystyrene plates (Wallac, part number 1450-510) in a final volume of 200 µl. All reagents were diluted into assay buffer (D-PBS containing 0.1% BSA (w/v)). The following mixtures were prepared:
- Biotin-PNA:: 1.5 ml assay buffer/0.75 µl Biotin-PNA stock
- Oligonucleotide:: 1 ml assay buffer/1.71 µl Oligo DNA stock
- ³H-labeled PNA (#1):: 0.75 ml assay buffer/6.9 µl ³H-labeled PNA stock (∼9000 cpm/well)
- ³H-labeled PNA (#2):: 0.733 ml assay buffer/17.4 µl ³H-labeled PNA stock (∼15,000 cpm/well)
- ³H-labeled PNA (#3):: 0.715 ml assay buffer/35 µl ³H-labeled PNA stock (∼38,000 cpm/well)
- Streptavidin beads:: 2.25 ml assay buffer/0.75 ml of 20 mg/ml stock

Reagents (50 µl each) were added to the well in the order listed above. For the specific conditions where a particular reagent was absent from the final incubation mixture, 50 µl of assay buffer was substituted. A 1 hour incubation at room temperature was performed prior to the addition of the streptavidin SPA beads to allow hybridization of the PNA probes to the oligonucleotide DNA. Once the SPA beads were added, a 30 minute incubation at room temperature was performed to capture the hybridized complex. SPA signal (CPM) was detected by counting the plates in a Wallac 1450 Microbeta microplate scintillation counter.

Table I below shows the means from a representative experiment performed in triplicate. A + indicates that the reagent was included in the incubation well and a - indicates that the reagent was omitted from the well and replaced with assay buffer. As was seen from the CPM (SPA counts per minute) columns, the maximum signal is only obtained when both PNA probes are present along with the oligonucleotide DNA (sample 4). In the absence of the biotin-PNA (sample 1 and sample 3), little signal was noticed because there was not a way to capture the signal to the streptavidin SPA bead. The maximum background was observed when the oligonucleotide DNA was absent in the assay (sample 2). This background signal may have been due to non-specific base pairing between the two PNA probes. The signal to noise ratio, defined by the signal for sample 4 divided by the signal for sample 2, increased as the level of radioactivity increased (see bottom line of Table I).

**Table I**

| Detection of Oligonucleotide DNA using SPA | | | | | | | |
|---|---|---|---|---|---|---|---|
| | | | | | **Level of Radioactivity** | | |
| **Sample** | **SPA Beads** | **3H-PNA** | **Biotin-PNA** | **Oligo DNA** | **#1 CPM** | **#2 CPM** | **#3 CPM** |
| 1 | + | + | - | - | 8 | 8 | 20 |
| 2 | + | + | + | - | 87 | 129 | 285 |
| 3 | + | + | - | + | 37 | 59 | 97 |
| 4 | + | + | + | + | 779 | 1514 | 3407 |
| | | | | | | | |
| Signal to noise Ratio | | | | | 8.9 | 11.7 | 12.8 |

A similar assay was performed in the presence of 0.1 mg/ml Torula Yeast RNA (5 Prime--3 Prime, Inc.) which minimized the non-specific interaction of the two PNA probes, as well as any non-specific interaction between the ³H-labeled PNA and the oligo DNA (sample 3 above).

The following mixtures were prepared for this experiment:
- Biotin-PNA:: 0.375 ml assay buffer/0.375 µl Biotin-PNA stock
- Oligonucleotide DNA:: 0.325 ml assay buffer/1.1 µl Oligo DNA stock
- ³H-labeled PNA :: 1.4 ml assay buffer/12.9 µl ³H-labeled PNA stock (∼5,500 cpm/well)
- Streptavidin beads:: 2.0 ml assay buffer/0.5 ml of 20 mg/ml stock
- Yeast total RNA:: 0.35 ml asay buffer/10.5 µl of 20 mg/ml stock

The assay was performed as described above. Table II shows the results of this assay. The volume (in µls) for each reagent added to the well is indicated in the table. The signal to noise ratio in the absence of added RNA was calculated by dividing the signal (CPM) obtained for sample 3 by the background signal with sample 2. Similarly, the signal to noise ratio in the presence of added RNA was calculated by dividing the signal for sample 8 by the appropriate signal in sample 7.

**Table II**

| Detection of Oligonucleotide DNA using SPA - Inclusion of Total RNA | | | | | | |
|---|---|---|---|---|---|---|
| | **50 µl** | **50 µl** | **50 µl** | **25 µl** | **25 µl** | |
| **Sample** | **SPA Beads** | **3H-PNA** | **Biotin-PNA** | **Oligo DNA** | **Total RNA** | **CPM** |
| 1 | + | + | - | - | - | 10 |
| 2 | + | + | + | - | - | 65 |
| 3 | + | + | + | + | - | 692 |
| 4 | + | + | - | + | - | 14 |
| 5 | + | + | + | - | + | 12 |
| 6 | + | + | - | - | + | 6 |
| 7 | + | + | - | + | + | 21 |
| 8 | + | + | + | + | + | 643 |
| Signal to Noise (- Total RNA) | | | | | | 10.6 |
| Signal to Noise (+ Total RNA) | | | | | | 30.6 |

This data shcwed that the inclusion of total RNA reduced non-specific association of the PNA probes (compare results for sample 2 versus sample 7) without significantly reducing the specific signal (compare sample 3 with sample 8). The addition of total RNA resulted in a 3-fold increase in signal to noise ratio with only a 7.1% reduction in overall signal.

The sensitivity demonstrated in this example can be determined by varying the amount of added oligonucleotide DNA while keeping other reagents (SPA beads, ³H-PNA and biotin-PNA) at constant levels. Reagent mixtures prepared for this experiment were:
- Biotin-PNA:: 6 ml assay buffer/3 µl Biotin-PNA stock
- Oligonucleotide DNA:: 0.9 ml assay buffer/1.73 µl Oligo DNA stock, then prepare 2-fold serial dilutions by diluting 0.45 ml of each into 0.45 ml of assay buffer
- ³H-labeled PNA :: 1.5 ml assay buffer/69.6 µl ³H-labeled PNA stock (∼33,000 cpm/well)
- Streptavidin beads:: 6.0 ml assay buffer/1.5 ml of 20 mg/ml stock

50 µl each of oligonucleotide DNA dilution, biotin-PNA and ³H-labeled PNA were added to the microtiter wells. Following a 1 hr incubation at room temperature, 50 µl of streptavidin SPA beads was added and the incubation was continued for 30 min at room temperature. SPA CPM were determined in a Wallac 1450 Microbeta. Mean values for triplicate determinations are shown in Table III.

**Table III**

| Sensitivity of Oligonucleotide DNA Detection | | | |
|---|---|---|---|
| **pmol DNA** | **SPA CPM** | **SD** | **Signal to Noise** |
| 3.75 | 3480 | 78 | 14.9 |
| 1.875 | 3675 | 77 | 15.8 |
| 0.9375 | 2837 | 8 | 12.2 |
| 0.46875 | 1395 | 170 | 6.0 |
| 0.23438 | 900 | 2 | 3.9 |
| 0.11719 | 538 | 3 | 2.3 |
| 0.05859 | 371 | 35 | 1.6 |
| 0.0293 | 305 | 35 | 1.3 |
| 0.01465 | 315 | 14 | 1.4 |
| 0.00732 | 279 | 23 | 1.2 |
| 0.00366 | 254 | 5 | 1.1 |
| 0 | 233 | 1 | 1.0 |

SD represents the standard deviation for triplicate determinations. This data indicated that the minimum detection level of the assay (defined by a minimum signal to noise ratio of 2.0) was approximately 100 femtomols of oligonucleotide DNA. When the amount of added oligonucleotide DNA exceeded the amount of biotin-PNA which can base pair with it and be captured by the streptavidin SPA bead, the signal decreased. The excess oligonucleotide DNA then competed with the DNA that was bound to the bead. This can be seen from the first data point listed in Table III.

### EXAMPLE 2

### Detection of mRNA using Fluorescein-labeled PNA (F-PNA) and Fluorescence Polarization Detection

The embodiment illustrated above was used to detect in-vivo generated mRNA using two separate F-PNA fragments custom ordered from PerSeptive Biosystems. The two F-PNA fragments were designed to have complementary base sequence to two separate regions at the 5' end of the leptin mRNA. Two distinct F-PNA fragments were used (a) to investigate if the region of mRNA would influence the assay, and (b) to enhance the assay sensitivity if necessary. The fluorescein label was covalently attached to the 5'-position in both PNA probes as shown below. A portion of the leptin mRNA (-450 bases in length) was synthesized in-house to test the assay concept. All experiments were carried out at room temperature.

| **Reagent** | **Sequence** |
|---|---|
| F-PNA, Lot CRD 4318: | 5'-Flu-ATT-GAT-CCT-GGT-GAC-AAT-NH2-3' (Molecular weight = 5273.3) |
| F-PNA, LOT CRD4319: | 5'-Flu-ACT-GCC-AG(W)-GTC-TGG-TCC-NH2-3' |
| | [W = 50%T, 50%A] |
| | (Molecular weight = 5220.5) |

Leptin mRNA fragment:
GGGGCATATG AGGGTGCCCA TCCAAAAAGT CCAAGATGAC ACCAAAACCC 50
TCATCAAGAC AATTGTCACC AGGATCAATG ACATTTCACA CGCGCAGTCA 100
GTCTCCTCCA AACAGAAAGT CACCGGTTTG GACTTCATTC CTGGGCTCCA 150
CCCCATCCTG ACCTTATCCA AGATGGACCA GACACTGGCA GTCTACCAAC 200
AGATCCTCAC CAGTATGCCT TCCAGAAACG TGATCCAAAT ATCCAACGAC 250
CTGGAGAACC TCCGGGATCT TCTTCACGTG CTGGCCTTCT CTAAGAGCTG 300
CCACTTGCCC TGGGCCAGTG GCCTGGAGAC CTTGGACAGC CTGGGGGGTG 350
TCCTGGAAGC TTCAGGCTAC TCCACAGAGG TGGTGGCCCT GAGCAGGCTG 400
CAGGGGTCTC TGCAGGACAT GCTTTGGCAG CTGGACCTGT CTCCCGGGTG 450
CTAATAGGAT 460 (SEQ ID NO:2)

SEQ ID NO:2 shown above, represents a portion of the leptin mRNA, and was synthesized using Ribomax™ Large scale RNA Production Kit obtained from Promega Corporation and an approriate DNA template. The F-PNA probes shown above contained complementary sequences to two distinct regions in this synthetic mRNA fragement. A negative control mRNA fragement that did not contain a complimentary reqion to SEQ ID NO:2 was also synthesized to demonstrate the specificity of the F-PNA probes.

### Preparation of PNA and mRNA reagents:

One micromole (1.0 µmole) of each F-PNA lot was received from the supplier as a lyophilized powder. Both lots were dissolved in 1.0 ml of 0.1% TFA in sterile water (v/v) to give a 1000 nmol/ml stock solution. Working solutions of 1.0 nmol/ml (1.0 pmol/µl) F-PNA were made in Dulbecco sterile PBS (D-PBS, Cat # 14080-022, Lot# 21N5447), pH 7.5. Stock and working solutions were stored at -40°C. Stock solution of leptin mRNA (∼75 mg/ml in sterile water, ∼ 135,000 g/mol, 55 µM) were used directly in all assays (55 µM = 55 pmol/µl).

### Fluorescence Polarization Assay:

This Example demonstrated an embodiment of the invention in which fluorescence polarization measurements are used to quantitate the bound reporter probes using a Beacon™ instrument (PanVera Corp., Madison, WI). The assay was performed in 12 x 75 mm borosilicate glass tubes using a total assay volume of 1.20-1.40 ml. In all cases the D-PBS, pH 7.5 was used as the reaction medium. The Beacon™ instrument was operated using the following settings: single blank subtraction, PMT voltage= 80, FP factor = 0.9950, heater off, lamp feedback on, blank and sample delay = 0 seconds.

### Experiment 1:

The polarization values (in milli-Polarization units, mP units) and the fluoresecence intensity values for the F-PNA lot CRD 4318 were measured in the D-PBS buffer. The polarization values measured for the F-PNA probes was shown to be in the range of 100-150 mP units, allowing a range of 350-400 mP units increase in singal when the probes bind to target mRNA:
- Buffer blank:: 1.20 ml D-PBS, pH 7.5 for CRD 4318
- Sample tube S1:: 1.20 ml D-PBS + 200 µl of 1 pmol/µl of CRD 4318 F-PNA
- Sample tube S2:: 1.20 ml D-PBS + 100 µl of 1 pmol/µl of CRD 4318 F-PNA
- Buffer blank:: 1.20 ml D-PBS, pH 7.5 for CRD 4319
- Sample tube S3:: 1.20 ml D-PBS + 200 µl of 1 pmol/µl of CRD 4319 F-PNA
- Sample tube S4:: 1.20 ml D-PBS + 100 µl of 1 pmol/µl of CRD 4319 F-PNA
The results of Experiemnt 1 (n=3) are shown in Table IV:

**Table IV**

| **Sample ID** | **Intensity IV** | **Intensity IH** | **Intensity IT** | **Polarization mP** |
|---|---|---|---|---|
| BufferBlank | 3435 | 1256 | 5935 | --- |
| CRD 4318 | 315584 | 248493 | 810086 | 121.4 |
| 200 pmols | (± 1449) | (± 908) | (± 3246) | (± 0.605) |
| CRD 4318 | 169862 | 132867 | 434267 | 124.7 |
| 100 pmols | (±1607) | (± 833) | (± 2811) | (± 4.18) |
| Buffer blank | 3525 | 1357 | 6227 | --- |
| CRD 4319 | 1149377 | 873118 | 2886880 | 139.1 |
| 200 pmols | (± 24863) | (± 21756) | (± 66108) | (± 1.82) |
| CRD 4319 | 634125 | 481050 | 1591413 | 139.8 |
| 100 pmols | (± 12489) | (± 11212) | (± 34801) | (± 1.82) |

These data demonstrated that the polarization values of the probes in D-PBS are in the range expected (100-150 mP units), based on their molecular weight of - 5300 g/mol. It was also noted that the standard deviations measured for the probes were very small (CV < 5%), refecting the inherent precision of polarization measurements. Hence, polarization measurements offered an added advantage in high throughput screening assays in 96-well formats. Fluorescence intensities measured for CRD 4319 F-PNA were approximately three times larger than that for CRD 4318, for the same concentration of probes. This was probably due to non-specific interaction or self-hybridization between molecules of CRD4319. This was also reflected in slightly higher value of polarization for the latter probe.

### Experiment 2:

The specific binding of F-PNA (CRD4318) to leptin mRNA fragment was demonstrated. A time-course experiment was carried out by adding 550 pmols of leptin mRNA and 110 pmols of control mRNA fragements to various amounts of F-PNA (CRD 4318) in D-PBS buffer.
- Buffer blank:: 1.20 ml D-PBS, pH 7.5
- Sample tube 1:: 3.70 pmols of F-PNA (CRD4318) + 550 pmols Leptin mRNA in 1.20 ml D-PBS
- Sample tube 2:: 1.23 pmols of F-PNA (CRD4318) + 550 pmols Leptin mRNA in 1.20 ml D-PBS
- Sample tube 3:: 0.111 pmols of F-PNA (CRD4318) + 550 pmols Leptin mRNAin 1.20 ml D-PBS
- Sample tube 4:: 3.70 pmols of F-PNA (CRD4318) + 110 pmols control mRNA in 1.20 ml D-PBS
- Sample tube 5:: 1.23 pmols of F-PNA (CRD4318) + 110 pmols control mRNA in 1.20 ml D-PBS
- Sample tube 6:: 0.111 pmols of F-PNA (CRD4318) + 110 pmols control mRNA in 1.20 ml D-PBS
The results from Experiment 2 are shown in Table V:

**Table V**

| Polarization (in mP units) | | | | | | |
|---|---|---|---|---|---|---|
| **Time (mins)** | **Leptin mRNA 550 pmoles** | **Leptin mRNA 550 pmoles** | **Leptin mRNA 550 pmoles** | **Control mRNA 110 pmoles** | **Control mRNA 110 pmoles** | **Control mRNA 110 pmoles** |
| | **+** | **+** | **+** | **+** | **+** | **+** |
| | **3.70 pmoles F-PNA** | **1.23 pmoles F-PNA** | **0.111 pmoles F-PNA** | **3.70 pmoles F-PNA** | **1.23 pmoles F-PNA** | **0.111 pmoles F-PNA** |
| 0.0 | 173.8 | 163.1 | 163.0 | 159.8 | 155.5 | 151.4 |
| 12.0 | 177.2 | 174.0 | 181.7 | 162.2 | 157.7 | 161.6 |
| 29.0 | 188.3 | 184.9 | 183.9 | 164.0 | 160.0 | 168.8 |
| 51.0 | 204.3 | 197.6 | 200.1 | ND | ND | ND |
| 77.0 | 204.8 | 206.0 | 210.2 | 165.5 | 160.3 | 152.9 |
| 111.0 | 212.5 | 213.8 | 219.9 | ND | ND | ND |
| 696.0 | 230.0 | 227.5 | 214.6 | ND | ND | ND |
| 771.0 | 242.5 | 244.9 | 245.0 | 172.0 | 165.2 | 165.2 |
| ND = Not determined | | | | | | |

Data clearly demonstrated that the leptin mRNA fragment specifically bound to the F-PNA probe. Polarization signals obtained using control mRNA fragment remained near the baseline values indicating the probe did not bind to mRNA fragments that did not contain the complementary base sequence. The small increase observed for control mRNA was probably due to non-specific binding.

Note that the polarization signal obtained using three different amounts of F-PNA probes were in excellent agreement during the entire time course of the experiment. This occurred because the experiment was designed using excess leptin mRNA (and control mRNA), to drive the equilibrium toward the formation of the complex, and to consume all the F-PNA probe in solution. Hence the net change in signal for leptin mRNA demonstrated the dynamic range under the conditions employed in this Experiment 2. These data also showed that the amount of F-PNA used in the assay can be decreased significantly without any effect on the observed polarization signal, and the signal was stable for well over 10-12 hours.

## Claims

1. A method for quantitating genetic material which comprises:
a) placing in an aqueous suspension of genetic material a peptide nucleic acid (PNA) or deoxy nucleotide guanidine (DNG) reporter probe tagged with a reporting group and a PNA or DNG capture probe tagged with a first member of a binding pair such that each probe is in molar excess of the genetic material to be quantitated under conditions that permit hybridization;
b) exposing the suspension in a) to a capture surface impregnated with a scintillant and tagged with a second member of the binding pair in a) under conditions that permit the binding pair members to bind to each other; and,
c) quantitating the amount of reporter probe that is complexed with the capture probe, the capture surface, and the genetic material to be measured.

2. The method of **Claim 1** wherein the reporter probe is a PNA reporter probe and the capture probe is a PNA capture probe

3. The method of **Claim 1** wherein the reporter probe is tagged with a radionuclide selected from the group consisting of ³H, ¹²⁵I, ¹⁴C, ³⁵S, ³²P, and ³³P.

4. The method of **Claim 1** wherein the reporter probe is tagged with ³H.

5. The method of **Claim 1** wherein the binding pair is selected from the group consisting of antigen:antibody, hapten:antibody, biotin:avidin, biotin:streptavidin, and biotin:neutravidin.

6. The method of **Claim 1** wherein the binding pair is biotin:streptavidin.

7. The method of **Claim 1** wherein the genetic material to be quantitated is single stranded DNA or RNA.

8. The method of **Claim 1** wherein the genetic material to be quantitated is mRNA encoding human leptin or SEQ. ID NO:2.

9. The method of **Claim 1** wherein the radionuclide is quantitated using scintillation proximity assay detection methodologies.

10. The method of **Claim 1** adapted for use in high volume screening wherein the reporter probe is an 18 base pair PNA reporter probe, the capture probe is an 18 base pair PNA capture probe, the reporter probe is tagged with ³H, the binding pair is biotin:streptavidin, the genetic material to be quantitated is mRNA encoding human leptin, and the radionuclide is quantitated using scintillation proximity assay detection methodologies.

11. A method for quantitating genetic material which comprises:
a) placing in an aqueous suspension of genetic material a PNA or DNG reporter probe in molar excess of genetic material to be quantitated tagged with a fluorophore under conditions that permit hybridization;
b) quantitating the amount of reporter probe that has hybridized with the genetic material to be measured using fluorescence polarization methodology.

12. The method of **Claim 11** wherein the reporter probe is a PNA reporter probe.

13. The method of **Claim 11** wherein the fluorophore is fluorescein.

14. The method of **Claim 11** wherein the genetic material to be quantitated is single stranded DNA or RNA.

15. The method of **Claim 11** wherein the genetic material to be quantitated is mRNA encoding human leptin or SEQ. ID NO:2.

16. The method of **Claim 11** adapted for use in high volume screening wherein the reporter probe is an 18 base pair PNA reporter probe tagged with fluorescein, and the genetic material to be quantitated is mRNA encoding human leptin.


SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: METHOD FOR MEASURING GENETIC MESSAGES

(iii) NUMBER OF SEQUENCES: 2

(iv) CORRESPONDENCE ADDRESS:
(A) ADDRESSEE: C. M. Hudson
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER: 96308898.4
(B) FILING DATE: 9th December 1996

(2) INFORMATION FOR SEQ ID NO:1:

(i) SEQUENCE CHARACTERISTICS:
(A) LENGTH: 52 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:1:

CAAAAAAACC ATCAGCAAGC AGGTATTGTT TCAACATGGC CCTGTGGATG CG
52

(2) INFORMATION FOR SEQ ID NO:2:

(i) SEQUENCE CHARACTERISTICS:
(A) LENGTH: 460 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:2:

GGGGCATATG AGGGTGCCCA TCCAAAAAGT CCAAGATGAC ACCAAAACCC
TCATCAAGAC 60

AATTGTCACC AGGATCAATG ACATTTCACA CGCGCAGTCA GTCTCCTCCA
AACAGAAAGT 120

CACCGGTTTG GACTTCATTC CTGGGCTCCA CCCCATCCTG ACCTTATCCA
AGATGGACCA 180

GACACTGGCA GTCTACCAAC AGATCCTCAC CAGTATGCCT TCCAGAAACG
TGATCCAAAT 240

ATCCAACGAC CTGGAGAACC TCCGGGATCT TCTTCACGTG CTGGCCTTCT
CTAAGAGCTG 300

CCACTTGCCC TGGGCCAGTG GCCTGGAGAC CTTGGACAGC CTGGGGGGTG
TCCTGGAAGC 360

TTCAGGCTAC TCCACAGAGG TGGTGGCCCT GAGCAGGCTG CAGGGGTCTC
TGCAGGACAT 420

GCTTTGGCAG CTGGACCTGT CTCCCGGGTG CTAATAGGAT 460